# EUROPEAN PATENT SPECIFICATION

(11) **EP 0 877 546 B1**
(45) Date of publication and mention of the grant of the patent: **16.08.2001**
(21) Application number: 96944605.3
(22) Date of filing: 16.12.1996
(51) Int. Cl.: A01H 4/00, A01H 1/04

(54) **PROCESS FOR PROPAGATION OF PLANT MATERIAL**
VERFAHREN ZUR VERMEHRUNG VON PFLANZENMATERIAL
PROCEDE DE MULTIPLICATION DE PLANTES

(30) Priority: 22.12.1995 EP 95309401; 16.08.1996 EP 96306008
(43) Date of publication of application: 18.11.1998
(73) Proprietor: SHELL INTERNATIONALE RESEARCH MAATSCHAPPIJ B.V., 2596 HR Den Haag (NL)
(72) Inventor: EDWARDS, Glyn, Alyn, Faversham Kent ME13 8NW (GB); PURSE, John, Gilbert, Newnham Sittingbourne Kent ME9 0JX (GB)
(86) International application number: EP9605746
(87) International publication number: WO9723126

(56) References cited:
- US-A- 4 569 914
- DATABASE WPI Section Ch, Week 9624 Derwent Publications Ltd., London, GB; Class C06, AN 96-233218 XP002031755 & JP 08 089 113 A (OJI PAPER CO) , 9 April 1996
- DATABASE WPI Section Ch, Week 9540 Derwent Publications Ltd., London, GB; Class C06, AN 95-307046 XP002031756 & JP 07 203 790 A (OJI PAPER CO) , 8 August 1995
- PLANT & CELL PHYSIOL., vol. 22, no. 3, 1981, pages 461-467, XP000671622 SHINSAKU TAKAYAMA ET AL.: "Mass propagation of Begonia x hiemalis Plantlets by shake culture"
- DATABASE WPI Section Ch, Week 9208 Derwent Publications Ltd., London, GB; Class C06, AN 92-060459 XP002031757 & JP 04 004 828 A (OJI PAPER CO) , 9 January 1992
- PATENT ABSTRACTS OF JAPAN vol. 018, no. 351 (C-1220), 4 July 1994 & JP 06 090631 A (KYOWA HAKKO KOGYO CO LTD;OTHERS: 01), 5 April 1994,
- BIOTECHNOLOGY AND BIOENGINEERING, vol. 34, no. 9, 1989, pages 1209-1213, XP000673298 JONG M. PARK ET AL.: "Cultivation of Artemisa annua L. Plantlets in a Bioreactor containing a single carbon and a single nitrogen source"

## Description

The present invention relates to a process for the vegetative propagation of plant material.

In vitro vegetative propagation (micropropagation) techniques are widely used for commercial-scale production of plants. Those techniques have the potential advantage over other methods of vegetative propagation, for example, propagation via cuttings, that a larger number of plants can be produced in a given time period. Furthermore, the techniques are useful for propagating genetically manipulated plant material. In the case of some plant genotypes, micropropagation techniques may be the only method available for vegetative propagation. However, although widely used in horticulture, commercial use of micropropagation techniques for the propagation of woody plants, for example, trees, is not widespread.

The vast majority of micropropagation techniques for vegetative propagation of woody plants use gelled growth media or use other physical means to support the plant material in or on a liquid growth medium. (Gelled media are often called "solid" whether they are in fact solid or semi-solid. Unless specified otherwise, the terms "solid media" and "semi-solid media" as used herein include both solid and semi-solid forms of gelled media.) However, micropropagation techniques that employ solid media or physical supports have the disadvantage that they are generally labour intensive and expensive. In addition, micropropagation techniques for woody plants often suffer from the disadvantage that it is difficult to produce uniform products. The reason for this is that it is often difficult to induce existing meristems, for example, axillary buds, to develop uniformly. That may be due to the meristems being quiescent or dormant and/or due to non-uniform elongation and development once growth has started. For commercial production, uniformity of product is extremely important. Any developmental heterogeneity of shoots set for rooting is often amplified during subsequent development of the plants.

There are therefore clear incentives to develop alternative micropropagation techniques for woody plants that are more efficient, more cost-effective and capable of producing a more uniform product.

It has been proposed to use submerged liquid media for micro-propagation as there are a number of potential advantages over solid media, including a greater ability to automate and hence to reduce expenses. Submerged culture of plant tissues has been employed extensively for cell culture i.e. growth of single cells, small groups of undifferentiated cells and embryogenic tissues or meristematic tissues. However, attempts to use liquid media to propagate shoots and plantlets has been hampered by a number of problems, and it appears that the technique has had only very limited application, to certain specific plants.

Hyperhydricity of shoots is a problem often encountered in liquid micropropagation systems, see for example, Aitken-Christie et al 1994 and George E F 1993/1996.

Hyperhydricity was previously termed vitrification (water-logging), but that is not technically correct. Hyperhydricity refers to the condition of in vitro cultured material that has an abnormal morphological appearance and physiological function. Susceptibility to hyperhydricity is not the same in all plants. Typical symptoms are shoots with shorter inter-nodes, brittle, curled or translucent leaves, abnormal anatomy including large intercellular spaces, reduced vascular system and surface wax; poorly developed stomata and chloroplasts; and altered biochemical characteristics including reduced lignin and cellulose and altered enzyme activities.

Virtually all plants cultured in vitro, even when grown on semi-solid media, show to some extent some of the symptoms associated with hyperhydricity. However, visual symptoms are irrelevant if those symptoms are mild and can be reversed easily. In such cases, the mild symptoms may not adversely affect plant propagation. The material under propagation can be recovered i.e. rooted, and the subsequent performance and hence value of the propagules is not affected adversely.

Hyperhydricity may cause problems in plant propagation when extreme expression of the characteristic symptoms described above is encountered. Hyperhydric shoots that show extreme expression of the characteristic symptoms under in vitro propagation conditions generally become difficult to propagate and may die whilst still in culture. They do not normally produce adventitious roots or root only poorly. Hyperhydritic shoots rarely survive transfer from an in vitro environment, for example, to the greenhouse. Such shoots are therefore generally unsuitable for rooting and/or subsequent propagation.

Hyperhydricity is most often encountered in the propagation of woody species. In woody plants, hyperhydricity of shoots and plantlets is a widespread problem and may be severe. Indeed, many woody plants are prone to severe hyperhydricity even when grown on solid or semi-solid growth media.

Park et al in Biotechnology and Bioengineering Vol 34, 1209 1213, 1989, describe cultivation of plantlets of Artemisia annua, an annual herbaceous plant, in submerged liquid culture. However, the plantlets produced were not tested for their ability to develop into plants, a crucial step in micropropagation, and Park indicates the need for further studies in relation to vitrification. In a later paper, 1993 Biotechnology Techniques 7; (10):697-702, 1993, Park with co-author Woo describes a mist culture system they developed and its use for cultivating Dianthus caryophyllus. Referring to his 1989 paper Park states that because of large size and shear sensitivity, it has been reported difficult to cultivate organized tissues in a liquid medium. Park also states that since serious vitrification of shoots takes place in liquid medium, acclimation steps of the shoots may be time-consuming or even unsuccessful.

Woerdenbag et al Plant Cell, Tissue and Organ Culture 32:247-57 (1993) grew shoots of Artemisia annua. The authors refer to the Park 1989 article but they themselves did not use the submerged culture method described by Park in 1989. Instead they used conventional shaken flasks to culture the Artemisia shoots.

Severe hyperhydricity is an extremely common phenomenon in liquid micropropagation systems. For that reason, liquid systems are generally avoided by the use of solid or semi-solid supports, for example, gelled media. In most of the cases where a liquid system is used, careful precautions are taken to avoid complete submersion of the plant material in the liquid culture medium.

A number of propagation techniques have been developed in which shoots or plantlets are floated on a liquid medium or grown in non-submerged (shallow) liquid media systems or in systems in which they are periodically flooded with culture medium. Gupta et al (Plant Sci Lett 20: 195-201, 1981) describe a method for multiplying and rooting seedlings and mature clones of Eucalyptus citriodora using protocols that have both solid and semi-submerged liquid culture media stages. However, such techniques have only limited advantages compared to techniques employing solid or semi-solid media.

Attempts have been made to reduce hyperhydricity in liquid culture by using growth retardants such as paclo-butrazol and ancymidol, which reduce the growth of the worst affected tissue, i.e. leaves. However, the use of growth retardants has been successful only with species such as Gladiolus or Nerine that are propagated in vitro via tubers or corms.

The present invention provides a process for the micro-propagation of shoots, rooted shoots or seedlings of a woody plant, which comprises cultivating the shoots, rooted shoots or seedlings in an oxygenated, for example, aerated liquid culture medium, the shoots, rooted shoots or seedlings being submerged in the liquid medium. The medium is generally agitated or otherwise moving.

The shoots, rooted shoots or seedlings may be allowed to move in the liquid medium, for example, they may be allowed to move freely, for example, to tumble, for example, tumble freely, in the liquid medium. Alternatively, their movement may be restricted or otherwise impeded.

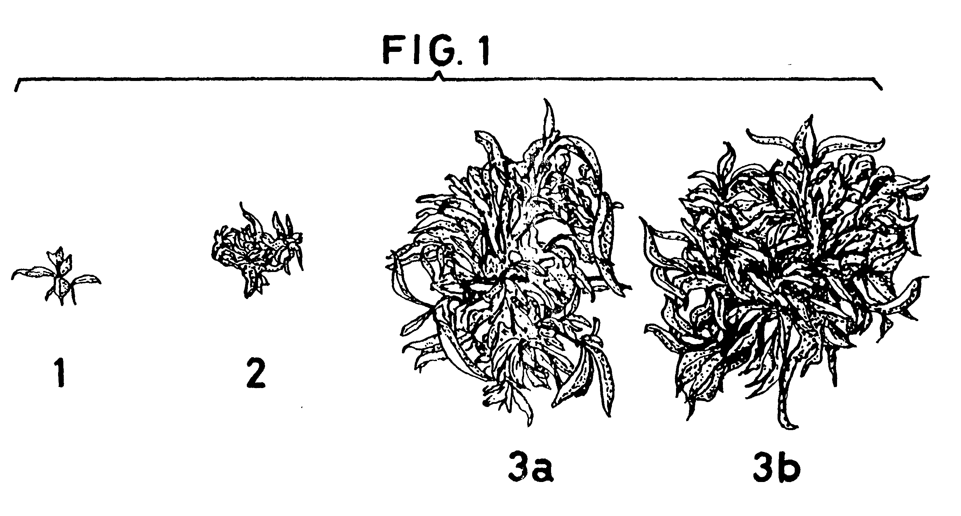

The process of the invention is simple, inexpensive in itself, reduces labour costs and gives excellent quality material that is suitable for large-scale commercial use, as shown in Figure 1 of the accompanying' drawings.

Figure 1 shows four shoot masses:
1: Typical micropropagated Eucalyptus grandis shoot used as inoculum.
2: Typical Eucalyptus grandis shoot mass produced after five weeks cultivation on solid micropropagation medium (KM medium).
3a,3b: Two typical Eucalyptus grandis shoot masses harvested after 27 days submerged liquid culture in liquid KM medium according to the present invention.

In the process of the present invention, the shoots, rooted shoots or seedlings are cultured under conditions such that they are completely immersed in the liquid medium. The shoots, rooted shoots or seedlings may be allowed to move in the liquid medium, for example, they may be allowed to move freely. For example, the shoots, rooted shoots or seedlings may tumble, for example, tumble freely, in the liquid medium. The tumbling may be vigorous and may be substantially continuous. Alternatively, the movement of the shoots, rooted shoots or seedlings may be restricted or otherwise impeded. For example, the shoots, rooted shoots or seedlings may be restrained, for example, they may be held by perforated restraining means, for example, in a perforated container, for example, a cage or bag, within the plant growth vessel or within a separate section of the vessel that is in contact with the liquid medium.

The liquid medium in which the plant material is cultured must comprise sufficient oxygen to support the metabolism of the plant material. It is generally necessary to provide oxygen, usually in the form of air, and/or to illuminate the system such that oxygen is produced by photosynthesis.

The medium may be agitated by mechanical means, for example, by means of a mechanical device, for example, a paddle or a stirrer, for example, a magnetic stirrer, or the vessel containing the medium may be agitated, for example, shaken, vibrated or rotated. The means used for agitation, for example, shaking or stirring, may oxygenate the medium sufficiently to support the metabolism of the plant material. If not, oxygen, generally in the form of air, may be provided and/or the system may be illuminated such that oxygen is produced by photosynthesis.

The production of oxygen by photosynthesis may totally or in part provide the oxygen required by the culture. The light required for the production of oxygen by photosynthesis will generally cause movement of the liquid medium by convection.

The liquid medium may be both agitated and oxygenated by passing oxygen or, more usually, air, through the medium. Air circulation techniques, sometimes called "airlift" techniques, are particularly useful for providing simultaneous oxygenation and agitation in the process of the present invention. Liquid medium in an appropriate vessel, for example, a fermentation vessel, for example, a flask, bottle, tank or column may be circulated and oxygenated by the introduction of air through, for example, a gas diffuser. Such vessels are often called "air-lift fermenters". The volume of air and the rate of introduction can easily be adjusted to give the desired degree of agitation to the liquid medium and hence to the plant material when it is allowed to move freely.

When the plant material is allowed to move freely, agitation of the medium by passing air or another oxygen-containing gas through the medium may be preferable to agitation by mechanical means, for example, stirring, because the shearing forces on the plant material may be lower. However, the shearing forces on the plant material in shaken or stirred systems of the present invention may by reduced by restricting the movement of the plant material, for example, as described in more detail below. In a system where air or another gas is passed through the medium it may also be desirable to restrict the movement of the plant material, so the liquid medium is agitated but the plant material is restrained.

If the passage of the gas is not sufficient to achieve the desired agitation, additional agitation means may be provided, for example, the plant growth vessel may be also be shaken or the contents may be stirred. The vessel may be illuminated to provide further oxygen by photosynthesis.

It should be noted that after inoculation in the liquid medium the shoots, rooted shoots or seedlings may not become wetted readily because air bubbles may bind to the surfaces. If so, the shoots, rooted shoots or seedlings may float at or near the surface of the medium. A surfactant may be included in the culture medium to assist wetting. Even in the absence of surfactant, however, the surfaces of the shoots, rooted shoots or seedlings will generally become thoroughly wetted within a few days. In an air-lift system, if there is sufficient agitation and they are not restricted, they will generally tumble freely.

As indicated above, the shoots, rooted shoots or seedlings that are propagated according to the submerged liquid culture process of the present invention may be allowed to move freely in the liquid culture medium or their movement may be restricted or otherwise impeded.

The plant material may be physically restrained in a container within the plant growth vessel or the vessel may be divided into sections, one or more sections containing plant material. The restraining or dividing means should allow passage of the sufficient liquid medium to allow adequate oxygenation of the plant material. Perforated or mesh materials may be used to construct the restraining means, for example, there may be used a perforated metal or plastics material, a wire mesh or a fabric, for example, muslin. Alternatively, fresh oxygen-containing liquid medium may be passed through a plant growth vessel containing the plant material, either continuously or periodically, the plant material being submerged at all times.

The movement of the plant material may be restricted in any the liquid culture system of the present invention, including those where oxygenation is achieved by shaking or stirring the liquid medium, by passing air or another oxygen-containing gas through the liquid medium, by photosynthesis, or by any combination of thereof. Restricting the movement of the plant material may have the advantage of reducing the shearing forces on the material. In the case of stirred cultures, it is particularly preferable to restrain the plant material because it may be damaged by the stirrer if it is allowed to move freely.

The liquid medium used for the propagation may be any medium that is suitable for the propagation of the chosen shoots, rooted shoots or seedlings. It should generally include sources of carbon and of nitrogen, organic and inorganic salts as required, and appropriate phytohormones and/or plant growth regulators. Suitable growth media are known, and an approp-riate medium may be chosen for the particular woody plant to be propagated. Known solid media may be modified by the omission of the gelling or other solidification agent to give a liquid medium. It may also be appropriate to modify levels of one or more of the components of a known medium for use according to the present invention, for example, it may be possible to use reduced levels of phytohormones and/or plant growth regulators compared with levels used in corresponding solid media. The optimum level of any particular component or combinations of components may be determined by conventional methods.

Appropriate culture conditions should be used, for example, with regard to temperature and light. It may be preferable to illuminate the culture to provide endogenous oxygenation to supplement or even, in some cases, to replace exogenous oxygenation. If the culture undergoes autotrophic growth under light, the carbohydrate source in the medium may be omitted or reduced. In such cases, the supply of air or another oxygen-containing gas may be supplemented with carbon dioxide. Alternatively, if sufficient oxygen is provided to support the metabolism of the plant material, cultivation may be carried out entirely or partially in the dark.

Shoots, rooted shoots and seedlings produced under cultivation in the dark may be elongated in comparison with those produced under illumination. Elongation may be advantageous for subsequent manipulation, for example, for dissection of the shoot or seedling. Furthermore, elongated shoots of some plants, for example, E. grandis and hybrids thereof, often root more easily than short shoots. Accordingly, the liquid culture system of the present invention enables manipulation of lighting conditions for the manipulation of shoot quality, which is important in commercial micropropagation systems.

Cultivation may be continued until the desired increase in biomass and in multiplication rates is achieved. Shoots may be removed from the liquid culture medium before or after rooting has initiated or has taken place and then transferred to a solid rooting medium for root initiation and/or development. The rooting medium used may contain activated charcoal, if desired. Alternatively, initiation of rooting and/or subsequent root development may be performed in vivo using suitable organic and/or inorganic substrates, for example, vermiculite, compost, soil or peat.

Shoots, rooted shoots (plantlets) or seedlings used for inoculation may be obtained by micropropagation, or germinated and/or grown in a growth chamber or cabinet, greenhouse or outdoors. The shoots, rooted shoots or seedlings may be obtained from a cultivar, clone or seed, especially from genetically valuable cultivars, clones or seed, or from genetically manipulated plant material. Shoots used for inoculation preferably have one or more nodes, for example, from two to four nodes. The shoot tip (apical meristem) may be present, or may be removed before inoculation. Removal of the apical meristem may result in a more uniform product.

Microbial contamination is a potential problem in liquid culture systems in general. In the present case, it may be a particular problem when the starting material has been obtained from plants grown under non-sterile conditions, for example, in a glasshouse or outdoors, even though surface sterilisation or disinfection is carried out according to conventional methods. Even when the starting material has been produced under sterile conditions there may be microbial contamination from other sources.

Accordingly, the liquid culture medium employed in the process of the present invention preferably comprises an antibiotic, for example, augmentin. However, the fact that the shoot, rooted shoot or seedling masses are totally immersed in the liquid medium should result in more efficient penetration of antibiotic into the tissues of the shoot masses than occurs on solid media, and hence the effect of microbial contamination, should it occur, is reduced.

This is particularly important for micropropagation according to the process of the present invention of genetically manipulated plant material obtained using Agrobacterium-mediated transfer. Such material may present particular problems for subsequent micro-propagation because of the inevitable contamination with the Agrobacteria themselves. Such problems are minimised in the process of the present invention.

Nevertheless, precautions are preferably taken to minimise the potential for contamination by exogenous microorganisms, for example, any supply of air or other gas used for oxygenation is preferably filtered, irradiated, chemically or otherwise treated to remove microorganisms; joints and connections are preferably sealed, and apparatus is preferably sterilised before use, for example, by autoclaving. Tissue culture grade materials, for example, tubing, are preferably used.

The process of the present invention is applicable to woody plants, that is to say, perennial plants that exhibit secondary growth (secondary thickening) of roots and/or aerial stems, which is the result of the formation of wood. Wood is secondary xylem, and is composed of one or more of the following: tracheids, vessels, fibres and rays. Woody plants include forest trees, other trees, shrubs and bushes.

Examples of woody plants that may be micropropagated by the process of the present invention include, but are not limited to, gymnosperms and dicotyledenous angiosperms, for example, as used for wood pulp, for fuel or for timber, for example, Eucalyptus, Pinus, Picea, Acacia, Populus, Betula, Tectona and tropical hardwoods; trees, shrubs and bushes that produce fruit or nuts, for example, apple, citrus, peach, olive, walnut and almond trees, coffee bushes, blackcurrant bushes, and raspberry canes; trees, shrubs and bushes from which other commercially useful products can be obtained, for example, rubber trees and trees and shrubs that produce pharmaceutically useful substances or precursors for pharmaceutically useful substances, for example, yew trees; and ornamental trees and shrubs, for example, trees and shrubs having ornamental flowers, foliage or bark.

Woody plants that appear to perform particularly well in the liquid culture system of the present invention are sclerophyllous species. The definition of sclerophyll is "thick, leathery leaf". This includes true leaves, as in the case of eucalypts and phyllodes as in the case of some Acacias. Species considered as sclerophyllous are generally evergreen and the sclerophyllous habit is generally associated with poor nutrient availability and often with drought tolerance. Examples of sclerophyllous genera are Rhododendron, Azalea and Kalmia (Ericaceae); Olea (Oleaceae); many Australian Acacias (Fabaceae); and eucalypts (Myrtaceae).

However, it is not only sclerophyllous species that perform well in the liquid culture system of the present invention. Malus (apple), Pyrus, Prunus and Rosa (Rosaceae), Forsythia and Syringa (Oleaceae) are further examples of woody plant genera that may be propagated using the liquid culture system of the present invention.

As indicated above, eucalyptus is an example of a sclerophyllous woody plant that may be propagated according to the present invention. The sub-genus Eucalyptus symphyomyrtus contains many commercially useful species, for example, E, grandis, E. globulus, E. nitens, E. dunnii, E. saligna, E. camaldulensis, E. urophylla and hybrids thereof. Further commercially important Eucalyptus species include E, regnans, E. citriodora, E. fraxinoides and hybrids thereof.

According to a particularly preferred embodiment of the process of the present invention, shoots, rooted shoots or seedlings are propagated in a submerged liquid culture that is oxygenated and agitated by means of circulating air such that the shoots or plants tumble, preferably freely. In such a system, which is often called an "air-lift" system, compressed air (or other suitable gas) is fed into a plant growth vessel containing a liquid medium and the shoots, rooted shoots or seedlings. The air or other gas supplied is preferably humidified before it enters the plant growth vessel for example, by passage through water, especially distilled water.

According to further preferred embodiments of the invention, the shoots, rooted shoots or seedlings are propagated in a vessel that is shaken or stirred. It is particularly preferred to restrict the movement of the plant material, for example, in perforated container, for example, a cage or bag as described above. It may also be advantageous to restrict the movement of the plant material in a culture system of the invention where air is passed through the liquid medium.

As indicated above, it is preferable to ensure that all apparatus and all other materials used are sterile to minimise microbial contamination. Apparatus is preferably sterilised before use by autoclaving, and media are sterilied by autoclaving or filtration, where possible, and tissue culture grade materials are preferably used. All joints in the apparatus should be carefully sealed.

Where air or another gas is supplied to a plant growth vessel, filters are preferably provided in the inlet and outlet of the air supply to the vessel to maintain sterility within the vessel. The air supply may be passed through a filter, for example, an activated charcoal filter, to remove gaseous and/or volatile contaminants in the air supply. The filters used, particularly exhaust filters, are preferably hydrophobic as there is inevitable evaporation from the apparatus with the potential for condensation in the exhaust air stream. For long-term operation, it may be desirable to incorporate a condenser in the exhaust air stream to avoid build-up of condensation and potential microbial growth that may occur in filters, decreasing flow rates and possibly causing infection of the culture (filter "grow-through" phenomenon).

The plant growth vessel may be of any size and shape suitable for submerged liquid culture. Suitable vessels are well known for "air-lift" systems and for systems where the liquid medium is shaken or stirred. The vessel may be, for example, a flask, bottle, column or tank. The vessel may be of glass, metal or even a synthetic polymer, for example, polypropylene or polycarbonate. If the plant material is to be illuminated for photosynthesis, the vessel should allow the passage of light of the appropriate wavelength.

The liquid medium is introduced into the vessel, is preferably brought to the temperature at which cultivation will be carried out, the inoculum of shoots, rooted shoots or seedlings is added to the medium, and the apparatus is generally sealed. The plant growth vessel and its contents are maintained at an appropriate temperature, for example, from 20 to 30°C, with or without illumination.

In the case when air or another oxygen-containing gas is passed through the liquid medium in which the plant material is free to move, the supply is preferably adjusted to the maximum flow rate that gives a steady stream of bubbles such that the shoots, rooted shoots or seedlings are submerged and preferably tumble, and especially tumble freely, in the medium. The tumbling may be substantially continuous. In the case of systems in which the liquid medium is shaken or stirred, the shaking or stirring should be sufficient to oxygenate the medium while ensuring that the plant material is submerged.

An appropriate medium is chosen for the woody plant to be propagated, for example, KM medium (without gelling agent) may be used for the propagation of Eucalyptus grandis and hybrids thereof. Similarly, for other plants the gelling agent may be omitted from a solid or semi-solid medium previously used for the propagation of that plant.

In some cases it may be possible to use levels of phytohormones lower than are conventionally used in solid media, for example, in the propagation of Eucalyptus grandis according to the present invention, good results are obtained with 25% (or even less) of the conventional amount of BAP (6-benzylaminopurine).

When shoots of a woody plant comprising an apical meristem, for example, Eucalyptus grandis, are propagated according to the air-lift system of the present invention with the shoots tumbling freely in the liquid medium, the effects of correlative inhibition on axillary shoot meristems appear to be almost completely abolished. All the branch systems originating from existing nodes present at time of inoculation contain tertiary branches. Nodes of decreasing age resulting from extension growth from the apex of the inoculated shoot possess branch systems of decreasing complexity. The shoot tips of the original stems, the primary branches and the secondary branches from both media all yield shoot tips that are well elongated, possess thick and robust stems and two or more well spaced nodes. There is remarkable degree of uniformity between the harvested shoots, regardless of the number of nodes that the source branches possessed at harvesting. Almost all of the tertiary branches consist of a shoot tip (two leaves and meristem) and no nodes.

When shoots are propagated in a submerged liquid culture system of the present invention where their movement is restricted, for example, by being held in a perforated container, for example, a cage or bag, within the plant growth vessel, the resulting shoots display substantially the same characteristics as described above for shoots that are able to move freely. They display a high degree of uniformity and are of good quality. Furthermore, the multiplication rates are generally as high as those for the freely moving shoots. It appears that a high shoot inoculation density may lead to a high multiplication rate.

Contrary to expectation, symptoms of hyperhydricity (vitrification) of leaves, if they occur, are mild and are only apparent on well expanded, older leaves. Younger material (i.e. at the shoot tips and apical nodes) does not show any significant signs of hyperhydricity. It has been found that, should any signs of hyperhydricity be observed during use of the liquid culture technique of the present invention, such signs are generally not significant, are readily reversed during subsequent culture on solid medium, and hence have little or no effect on the subsequent propagation or rooting of the shoots.

Shoots harvested from the plant growth vessels root well and, for example in the case of E. grandis, rooting efficiency may be better than that resulting from methods of propagation that utilise solid media. In the case of Acacia mangium, the resulting shoots can be rooted in compost and transferred directly to the greenhouse without the need for rooting on solid media. This is a most surprising advantage, which is very important commercially. The quality of the Rhododendron and eucalyptus shoots and their good rooting efficiency indicates that they, too, may be rooted directly in compost and transferred to the greenhouse without an intermediate rooting stage in semi-solid media. Indeed, the good quality of the material obtained according to the process of the present invention suggests that direct rooting in compost and transfer to the green house may be possible with other genera in addition to Acacia and Eucalyptus.

In summary, the process of the present invention yields an abundance of green, healthy, highly uniform shoots capable of rooting or further propagation. In many cases the shoots are sufficiently elongated that they can be rooted directly without further specific elongation steps. In some cases the shoots can even be rooted directly in compost and transferred to the greenhouse without an intermediate stage of rooting on semi-solid media.

Some plants, for example, olive, become chlorotic (yellow) when propagated on gelled media. In contrast, when propagated according to the liquid culture system of the present invention, olive shoots are green and healthy. A further advantage is that the olive shoots are well elongated. In contrast, when propagated on gelled media, olive shoots require an extensive elongation period, usually of about a month, before rooting.

The extension rates of new shoots propagated using the liquid culture system of the invention appears to be unusually uniform. For commercial micropropagation, uniformity of product is extremely important. Any developmental heterogeneity of shoots set for rooting is often amplified during subsequent development of the plants. Such heterogeneity is usual in woody plants, resulting in the need for grading of products and possible interruptions of supply.

This problem is illustrated in the commercial production of rhododendrons. In all the gelled systems used for rhododendron production, large amounts of callus may be formed on the base of the multiplying shoots. Adventitious shoots are often produced from this callus via organogenesis. These adventitious shoots are frequently not true to type (i.e. are abnormal) due to the phenomenon of somaclonal variation. (This phenomenon is often associated with changes in chromosome number or chromosomal rearrangements.) Currently, commercial rhododendron producers have to sacrifice multiplication rates in order to avoid callus and adventitious shoot formation to ensure that the plants they are propagating are true to type. The liquid system of the present invention avoids this problem as the uniformity of the resulting shoots is excellent and far superior to that obtained using gelled propagation systems. In particular, no adventitious shoots are produced, all new growth resulting from development of axillary buds.

Furthermore, as indicated above, Acacia shoots can be rooted in compost and transferred directly to the greenhouse without the need for rooting on solid media. This surprising advantage is very important commercially.

There are also major cost advantages compared to propagation systems that use solid media, including savings in manual labour (or the high capital costs of automating processes using solid media), disposables and time. For instance, to produce 50 E. grandis shoots from a single starting shoot using typical solid media. protocol requires three subculture steps (and associated media preparation and use of disposable culture dishes) and takes three months. This compares to a single culture step and one month using the system of the present invention.

A further advantage has been found when propagating Acacia and Eucalyptus according to the present invention. Acacia shoots form in clumps that can be dissected out to give individual rooting shoots. The remains of the clumps, consisting of branched stems but without shoot tips, may be reintroduced into liquid culture medium according to the process of the present invention, whereupon further shoots develop. This process may be repeated several times at least, without deterimental effect on the quality or quantity of the product.

With Eucalyptus, shoots may be harvestec from a liquid system according to the invention, dissected from shoot masses and immediately reinoculated into plant growth vessels containing fresh media. The process may be repeated for several cycles at least, without any significant effect on the shoot multi-plication rates and without any deterioration in the quality of the shoots produced in successive cycles. It is considered that recycling of the propagation material in the process of the present invention will have general applicability, and may be used for other genera in addition to Acacia and Eucalyptus.

Recycling the propagation material results in considerable savings and a uniform product, so is particularly attractive commercially.

Thus, the system of the present invention is highly attractive commercially. The yields obtained are generally higher than those with gelled systems, and are often much higher. The high quality of the product, both in the general health of the product and its remarkable uniformity, is a particular commercial attraction. For some species there are further specific advantages, as described above.

The shoots, rooted shoots or seedlings obtained by any process of the invention described herein may be grown into plants, for example, mature plants, under appropriate conditions, for example, in a greenhouse or outdoors. It is an advantage that, in many cases, shoots obtained according to a process of the invention may be rooted directly into compost and grown up in a greenhouse, rather than requiring an intermediate stage of rooting on semi-solid media.

As set out above, depending on the embodiment of the invention, the plants may be, for example, annual, biennial or perennial plants; they may be gymnosperms, monocotyledonous or dicotyledonous plants; they may be herbaceous or woody plants, for example, the sclerophyllous and other woody plants specifically described above. The plants may of use in agriculture, horticulture, forestry or as plantation crops. The plants may be ornamental or produce useful crops or products. Examples of plants and their uses are given above.

Plants obtained from shoots, rooted shoots or seedlings obtained by any process of the present invention are themselves part of the present invention, as are products obtained from such plants. Such plants may be micropropagated according to a micropropagation process of the present invention. For the reasons given above, this is particularly useful for the propagation of mature plants, for example, mature eucalypts.

The following non-limiting Examples illustrate the invention.

### EXAMPLES 1 TO 10

### PROPAGATION OF E. GRANDIS AND E. GRANDIS HYBRIDS

Unless specified otherwise the media, plant materials, temperature, airflow and lighting conditions described below were used in the following Examples 1 to 10:

### Media

Solid KM media for micropropagation of E. grandis & E. grandis hybrids

| | |
|---|---|
| Phytagel | 3 g/l |
| Sucrose | 10 g/l |
| 10 X Macronutrient solution¹ | 100 ml/l |
| MgSO₄.7H₂O | 0.925 g/l |
| NH₄NO₃ | 0.825 g/l |
| Murashige and Skoog (1962)² basal salt | 50 ml/l |
| micronutrient stock solution (Sigma M0529) 1000 X Murashige and Skoog (1962) | 0.5 ml/l |
| vitamin solution (Sigma M03900) BAP (6-benzylaminopurine) | 0.04 mg/l |
| Adjust pH 5.6. with KOH and autoclave at 121 °C for 20 minutes. | |

| | |
|---|---|
| ¹10 X macronutrient solution contains 2.2 g/l CaCl₂.2H₂O, 0.85 g/l KH2P04 and 1.9 g/l KNO₃ | |
| ²Murashige T; Skoog F: (1962) A revised medium for rapid growth and assay with Tobacco tissue cultures. Physiol. Plant., 15 473-497. | |

Liquid KM media for micropropagation of E. grandis & E. grandis hybrids

Ingredients: as for the solid KM medium, except that
(i) the Phytagel is omitted and
(ii) 0.01 mg/l BAP are used instead of 0.04 mg/l BAP that is used in the solid medium.

Half-strength KM medium for rooting of shoots of E. grandis and E. grandis hybrids

This medium contains half the macronutrient, micronutrient, MgSO₄.7H₂O and NH₄NO₃ content of the basic solid KM medium. BAP is omitted and replaced with 0.2 mg/l IBA (indoyl-3-butyric acid). The pH is adjusted to pH 5.6 and the medium is autoclaved at 121 °C for 20 minutes as described above for the full strength basic KM medium.

Where applicable, 1.0 g/l activated charcoal is added prior to sterilisation.

### Temperature, airflow and lighting conditions

Liquid micropropagation, micropropagation and rooting on semi-solid media were conducted at 22 °C with a 16 hour photoperiod (50-70 µmol m⁻² s⁻¹, supplied by fluorescent lamps). Unless specified otherwise, airflow rates were in the range of from 0.3-0.7 litre per minute per litre of liquid medium.

### Plant material used for inoculation

All plant materials used for inoculation were previously micropropagated on semi-solid media. E. urophylla x grandis hybrid clones 18.50 and 18.52 were obtained from Centre National de Recherches Forestieres, B.P. 764, Pointe Noire, Republique Du Congo.

E. grandis clones 5046 and 5048 were obtained from Prof D.L. Rockwood, Dept. Forestry, Univ. Florida, Gainesville, Florida 32611-0303 USA.

E. grandis x E. camaldulensis hybrid clone 11/25 was supplied to Shell South Africa by the South African Forestry Research Institute, PO Box 727, Pretoria 0001, South Africa.

Micropropagated E. grandis seedling line T14 L10 was derived from seed of E. grandis supplied by the Institute of Commercial Forestry Research, University of Natal, P.O. Box 375, Pietermaritzberg 3200, Republic of South Africa (seed batch reference No. 38064).

### Preparation of shoots used for inoculation

Micropropagated shoots of E. grandis or E. grandis hybrids having apical meristems were used as starting material. Each shoot possessed 2-4 nodes and a shoot tip. The shoots had previously been serially subcultured on basic solid KM medium containing 0.04 mg/l BAP. The cultures from which the shoot tips were harvested had rooted spontaneously. The plant material from which the shoots were derived had previously been cultured for extensive periods on antibiotic (augmentin)-containing media and was known to be free of microbial contaminants.

### Multiplication rates of shoots

Unless specified otherwise, the multiplication (propagation) rates given in the Examples are for shoots that are suitable for rooting either directly into compost or indirectly via semi-solid media.

### EXAMPLE 1

### Propagation of E. grandis shoots using an airlift system with free movement of shoots.

The media, temperature, airflow and illumination conditions used were those set out above.

### Apparatus

The apparatus consisted a compressed air supply fed via a combined pressure regulator and gauge. A pressure release valve (NUPRO SS-6C-MM-10), activated when the pressure reaches approximately 10 p.s.i. (approximately 67,000 Pa) was fixed into the line downstream of the regulator. The airstream was passed through an activated charcoal gas filter (Whatman Carbon Cap 75) and humidified by passage through distilled water using a gas diffuser (Pyrex no. 2) and a 21 Erlenmayer flask. The deodorised and humidified air stream was used to supply an air-lift fermenter. The airstream was sterilised by passage through a gas filter (Whatman Hepa-Vent 0.3 µm pore size) before being fed into the air-lift fermenter. The air-lift fermenter comprised a 51 culture vessel (Quickfit FV) fitted with a lid (Quickfit MAF2/2) having five 19 mm ports. The airstream was passed through the central port using 6mm diameter stainless steel tubing and a lid adaptor (Quickfit). A gas diffusion tube (Pyrex no. 2) was attached to the stainless steel inlet so that the diffuser was approximately 0.5 cm from the base of the assembled culture vessel. The exhaust consisted of a stainless steel tube, inserted into a port using a screwjoint adaptor (Quickfit) vented to the atmosphere via a gas filter (Whatman Hepa-Vent).

Silicone tubing (tissue culture grade, Merck) was used throughout the apparatus downstream of the activated charcoal filter. It is possible to replace the glass culture vessels by plastics (tissue culture grade) bottles, for example, tissue culture grade polypropylene or polycarbonate bottles, which are considerably cheaper.

### Preparation of the apparatus

The air-lift fermenter vessel was filled with 4.51 of the liquid KM medium described above. Silicone grease was used to ensure a good seal of the flange and the lid secured using a springclip (Quickfit JC 100F). Two of the remaining ports were securely stoppered. The inlet, exhaust ports and two stoppered ports were wrapped in foil and sealed with tape as an added precaution against these becoming potential routes for contamination. The remaining port was loosely stoppered and loosely wrapped in foil and tape to allow access of steam during sterilisation and to enable subsequent inoculation with shoots. The fermenter and inlet/exhaust filters were autoclaved as assembled units using a 30 min preheat/-60 min full pressure (121 °C) cycle. The fermenter was allowed to cool to room temperature (overnight) before the inoculation port was secured and the fermenter removed from the autoclave. The fermenter was placed in a 22 °C waterbath, connected to the air supplies and equilibrated for 1 hour prior to inoculation (air-flow rate approximately 1.8 litres/minute).

### Micropropagation of shoots

On the first day, the fermenter was inoculated with ten micropropagated E. grandis shoots obtained from the seedling line T14 L10. The total weight of the inoculum was 493 mg. The fermenter was reconnected to the air supplies. The air-flow was adjusted to the maximum flow rate that gave a steady stream of bubbles, approximately 1.8 l/minute. The fermenter was shielded with safety screens, and operated for a period of 27 days prior to estimation of multiplication rates and increase of biomass.

Biomass increase was estimated by removing three shoot masses, blotting dry onto filter papers and weighing. Moisture content was estimated by drying to constant weight in a vacuum oven at 60 °C for 72 hours. The percentage moisture content of micropropagated shoots similar to those used for inoculation was estimated using the same method. Multiplication rates were estimated by dissection of three shoot masses from each fermenter into nodal and shoot tip explants. The nodal explants and some of the shoot tip explants were subcultured onto two solid basic micropropagation media (solid KM media containing 0.04 mg/l BAP and/or solid KM media containing both 0.04 mg/l BAP and 1% w/v activated charcoal). Rooting efficiencies of shoot-tip explants were estimated by transfer onto two rooting media (solid half-strength KM media containing 0.2 mg/l IBA and solid KM media containing both 0.2 mg/l IBA and 1% activated charcoal). After 5 days, some of the shoot tips that were initially transferred onto rooting medium containing activated charcoal were subcultured onto rooting medium lacking charcoal.

### Results

After initial inoculation, the surface of the shoots did not readily become wetted. Air bubbles bound to the shoots which floated on the surface of the media and did not tumble to any great extent. After several days, the surfaces of the shoots became wetted and the shoots began to tumble extensively.

Nine days after inoculation, the shoots were showing high rates of extension growth at the apices and the majority of the axillary buds that were present at the time of inoculation had commenced growth. A few of the larger leaves present at the time of inoculation were showing signs of hyperhydricity (vitrification), necrosis and undergoing abscission.

Sixteen days after inoculation, the medium was starting to become turbid. Some signs of hyperhydricity, necrosis and abscission of older leaves was becoming apparent. Side-shoots were continuing to elongate. The new axillary buds resulting from extension growth from the apices and primary branches were starting to commence growth. All of the new growth appeared healthy with no abnormal development being apparent. Six of the shoot masses were developing roots from the base of the shoot masses.

Twenty-four days after inoculation, turbidity of the medium had increased and a scum-like deposit had formed on the inside of the vessel above the level of the media. Signs of hyperhydricity, necrosis and abscission of older leaves became more pronounced and was occurring on the larger leaves of the primary branches. Samples of the media were taken and examined microscopically in order to determine the cause of the turbidity. The medium contained both intact and disrupted plant cells and also large amounts of cell debris, but there was no evidence of microbial contamination. Overall, the shoot masses were compact, green and showed few signs of hyperhydricity. A few of the new primary and secondary branches had broken off the main shoot masses.

The shoot masses were harvested from the fermenter 27 days after inoculation. Figure 1 shows the appearance of typical shoot masses from the fermenter in comparison with the typical shoot used as inoculum and with a typical shoot mass cultivated on solid media: The shoot mass 1 in Figure 1 is typical micropropagated shoot used as inoculum. The shoot mass 2 is a typical shoot mass produced after five weeks cultivation on solid micropropagation medium (KM medium). The shoot masses 3a and 3b are two typical shoot masses harvested after 27 days submerged liquid culture in liquid KM medium.

Estimates of increase in wet weight, dry biomass and moisture content of shoots harvested from fermenters are shown in Table 1 below.

**TABLE 1**

| Wet weight, dry biomass and moisture content | |
|---|---|
| - Wet-weight of inoculum (10 shoots) | 493 mg |
| - Estimated dry weight of inoculum (at 91.8% moisture content) | 40.4 mg |
| - Wet weight of 3 typical shoot masses harvested from fermenters | 15.466 g |
| - Estimated increase in wet weight (corrected for total yield of 10 shoot masses) | 104.5-fold |
| - Dry weight of three typical shoot masses harvested from fermenters | 1.671 g |
| - Estimated dry weight increase (corrected for total yield of 10 shoot masses) | 137.9-fold |

Estimates of total multiplication rates and shoot-tip multiplications rates are shown in Table 2 below. In that Table, estimates of micropropagation rates are expressed as shoot-tip explants which were judged suitable for directly setting for rooting and as total explants which were suitable for further micropropagation on solid media.

**TABLE 2**

| Multiplication rates | |
|---|---|
| Number of shoot-tip explants suitable for rooting from each of three shoot masses | 59, 46, 56 |
| Average | 54 |
| Estimated number of explants suitable for further micro-propagation from each of three shoot masses | 111, 102, 121 |
| Average | 111 |

In the resulting shoots, the effects of correlative inhibition on axillary shoot meristems appeared to be almost completely abolished. All the branch systems originating from existing nodes present at time of inoculation contained tertiary branches. Nodes of decreasing age resulting from extension growth from the apex of the inoculated shoot possessed branch systems of decreasing complexity.

Tertiary branches had started to form from axillary buds on the secondary branches. The main stems had grown extensively and the shoot masses possessed an average of 10 nodes (range 9-11 on the three shoots dissected). The stems of the original explants had expanded to approximately 2 mm diameter at the base and the primary, secondary and tertiary branches had decreasingly smaller diameter stems at their branch points. The shoot tips of the original stems, the primary branches and the secondary branches from both media all yielded shoot tips that were well elongated, possessed thick and robust stems and two or more well spaced nodes. Within a single branching class of shoots, there was a remarkable degree of uniformity between the harvested shoots, regardless of the number of nodes that the source branches possessed. Almost all of the tertiary branches consisted of a shoot tip (two leaves and meristem) and no nodes. The greatest difference between shoots of a single branching class was seen in the degree of elongation of the tertiary shoots. Only well elongated tertiary shoots were harvested for subsequent transfer to solid media. Roots were only observed developing from the base of seven of the main inoculated shoots.

Where observed, signs of hyperhydricity of leaves was apparent on well expanded, older leaves only and was not as advanced as was expected from observations made whilst the fermenter was in operation. Younger material (i.e. at the shoot tips and apical nodes) did not show any significant signs of hyperhydricity. Dissection of the shoot masses indicated that the older stems were rather brittle, indicating hyperhydricity.

After harvest from the shoot masses produced in liquid KM medium and subsequent transfer to solid KM micropropagation media, shoot-tip explants and nodal explants appeared healthy. Four days after transfer, no differences could be detected between explants transferred to KM micropropagation media containing or without activated charcoal. The explants looked healthy in comparison to similarly treated shoot-tip and nodal explants previously cultured on solid KM micropropagation medium lacking activated charcoal. 28 days after transfer, the growth of apical meristems (where present), commencement of growth of new axillary meristems and subsequent elongation growth of axillary shoots appeared to be similar in explants either harvested from shoot masses produced in liquid KM medium or in explants previously cultured on solid KM micropropagation medium lacking activated charcoal.

Rooting efficiencies of shoot-tip explants harvested either from shoot masses produced in liquid KM medium or harvested from shoots previously cultured on solid KM micropropagation medium lacking activated charcoal are shown in Table 3.

**TABLE 3**

| Rooting efficiencies of shoot-tip explants 28 days after transfer to rooting media Percentage rooting of shoot-tip explants | | |
|---|---|---|
| Rooting Treatment | Shoot-tip explants harvested from shoot masses produced in liquid KM medium | Shoot tip explants harvested from shoot cultures produced on solid KM medium lacking activated charcoal |
| Half-strength KM rooting medium | 86 | 66 |
| Half-strength KM rooting medium plus activated charcoal | 52 | 41 |
| Half-strength KM rooting medium: 5 days with activated-charcoal, 23 days lacking activated charcoal | 84 | 53 |

Under the three condition tested, rooting efficiencies of shoots-tip explants harvested from shoot masses produced in liquid KM medium were higher than for shoot-tip explants harvested from shoots previously cultured on solid KM micropropagation medium lacking activated charcoal. Rooting of shoots-tip explants harvested from the fermenter was first observed after 10 days transfer to solid rooting medium, two days earlier than shoot-tips harvested from shoots previously cultured on solid KM micropropagation medium lacking activated charcoal.

The micropropagation of E. grandis described above gave high multiplication rates and yielded an abundance of shoots for subsequent rooting. The shoots for rooting showed an increase of about 50 fold per month and the total number of explants that could be used for any subsequent micropropagation steps increased in excess of 100-fold per month. The high levels of multiplication were mirrored by the increase in biomass.

As described above, the quality of the shoots harvested from the shoot masses produced in liquid media was high, with few overt signs of hyperhydricity. The signs of hyperhydricity that were observed were confined to older tissues. The shoots harvested from the fermenters appear to root more readily on solid media containing IBA than control shoots harvested directly from cultures propagated on solid medium. The quality of the shoots is such that they may be suitable for direct rooting into compost in the greenhouse, as with the shoots of Acacia mangium described in Example 11 below.

### EXAMPLE 2

### Propagation of E. grandis hybrid clones & E. grandis seedlings

A number different E. grandis hybrid clones and the micropropagated E. grandis seedling line T14 L10 were micro-propagated using the air-lift method described in Example 1 except that commercially available polycarbonate vessels having a capacity of about 2.5 litres ("2 litre" vessels of Nalgene, Nalge Co. Box 20365, Rochester, NY 14602-00365, USA; cat. no. 2015-2000) and polypropylene screw tops containing inlet and outlet ports (Nalgene cat. no. 2162-0531) were used instead of the glass vessels.

The oxygenation/aeration system was fitted using a preformed (inlet) port as described in Example 1. 2.0 litre of liquid KM micropropagation medium as described above was placed in each vessel and the vessels were autoclaved (10 min preheat 20 min full pressure (12 °C) cycle). Once cooled, each vessel was connected to the air supply for equilibration for 1 hour prior to inoculation with 20 shoots of one of the clones or of the seedling line indicated, which clones and seedlings had previously been propagated on solid KM medium. Operation of the vessels was terminated after 22 days because some of the vessels had become full of propagating shoot masses.

Five typical shoot masses from each vessel were dissected in order to estimate the multiplication rates achieved. Table 4 show the multiplication rates obtained for the five clones and one seedling line used.

**TABLE 4**

| Multiplication rates of different E. grandis genotypes and hybrids in air-lift fermenters | |
|---|---|
| Shoot genotype | Multiplication rate (after 22 days) (av. of 5 shoots) |
| Seedling line T14 L10 | 24.4 |
| E. grandis x camaldulensis clone 11/25 | 24.7 |
| E. urophylla x grandis clone 18.50 | 31.3 |
| E. urophylla x grandis clone 18.52 | 30.7 |
| E. grandis clone 5046 | 11.7 |
| E. grandis clone 5048 | 3.1 |

For five of the six genotypes used, the multiplication rates are considerably higher than could be achieved using a semi-solid (gelled) medium system. In four of the six genotypes, multiplication rates are 8 to 10 times higher than could be achieved using a semi-solid (gelled) medium system.

### EXAMPLE 3

### Propagation of eucalyptus shoots using an airlift system with restrained shoots

The airlift system used in Examples 1 and 2 was modified by restraining the inoculated shoots in a 125 cm³ stainless steel cage containing perforations to allow the free passage of medium over the shoots instead of allowing the shoots to move freely in the liquid medium. A duplicate was carried out in which the shoots were unrestrained.

The method used was as described in Example 2 except that 250 ml polycarbonate containers (Nalgene cat. No. 2127-0250) and polypropylene screw tops containing inlet and outlet ports (Nalgene cat. no. 2162-0531) were used instead of the vessels previously described. The volume of medium used was 150 ml per vessel. Preparation and operation of the vessels was as described for the previous example.

Each vessel was inoculated with either 5 shoots of E. grandis x camaldulensis clone 11/25 or with 5 shoots of the micropropagated E. grandis seedling line T14 L10, the shoots having previously been propagated on solid KM medium cultures. In one set of vessels the shoots were allowed to move freely. Another set of vessels the shoots were restrained in a cage as described above. The shoots were cultured for 21 days then dissected to determine the multiplication rates achieved, which are shown in Table 5 below.

**TABLE 5**

| Shoot genotype | Multiplication rate (after 21 days) (av. of 5 shoots) | |
|---|---|---|
| | Unrestrained | Restrained |
| E. grandis x camaldulensis clone 11/25 | 8 | 10 |
| | | |
| E. grandis seedling line T14 L10 | 17.7 | 16.8 |

The multiplication rates achieved indicate that free movement of the shoots is not necessary to achieve high multiplication rates.

### EXAMPLE 4

### Propagation of eucalyptus shoots in a shaken flask system.

The method used was as described in Example 3 except that the gas diffusion tube was not present in the apparatus. The vessels were vented to the atmosphere using a single filter attached to an inlet/outlet port as previously described. The vessels were shaken at 125 r.p.m. on an Infors CH1043 shaker for 22 days. The shoots used were from the micropropagated seedling line T14 L10. Duplicate sets of vessels were run. In one set the shoots were not restrained; in the other they were restrained in a cage as described in Example 3.

The multiplication rates obtained were 2 for the unrestrained shoots and 4.2 for the restrained shoots. This result demonstrates that multiplication will occur in a shaken flask in which plant material is fully submerged.

### EXAMPLE 5

### Propagation of eucalyptus using a stirred system

The procedure described in Example 4 was carried out except that, instead of being shaken, the contents of the vessels were stirred using a 2.5 cm magnetic stirrer bar. The vessels were placed on magnetic stirrers adjusted to give a rotation rate of the stirrer bar of approximately 250 r.p.m. Each vessel was inoculated with either 5 shoots of E. grandis x camaldulensis clone 11/25 or with 5 shoots of the micropropagated E. grandis seedling line T14 L10. Duplicate sets of vessels were run; in one set the shoots were restrained in a cage as described in Example 3, in the other set the shoots were allowed to move freely. The results are given in Table 6 below.

**TABLE 6**

| Shoot genotype | Multiplication rate (after 22 days) (av of 5 shoot masses) | |
|---|---|---|
| | Unrestrained | Restrained |
| E. grandis x camaldulensis clone 11/25 | macerated | 8 |
| | | |
| E. grandis seedling line T14 L10 | macerated | 3.2 |

No usable shoots were recovered from the vessel containing the unrestrained shoots due to mechanical damage of the shoots caused by the magnetic stirrer bar.

### EXAMPLE 6

### Propagation of eucalyptus shoots in the light & in the dark

The procedure described in Example 2 i.e. using the airlift system was repeated except that 250 ml vessels were used and the shoots were from the micropropagated E. grandis seedling line T14 L10. The lighting conditions were as described at the beginning of this section i.e. 16 hour photoperiod light (50-70 µmol m⁻² s⁻¹ supplied by fluorescent lamps). A duplicate was carried out, but in continuous darkness instead of continuous light.

The multiplication rates obtained after 22 days culture were 24.4 for the vessel incubated in the light and 21.2 for the vessel incubated in the dark. This indicates that light is not essential in order to obtain high shoot multiplication rates provided oxygenation is adequate.

The shoots cultured in the dark were more elongated than those produced in the light, indicating that manipulation of lighting conditions can be used to manipulate shoot quality, which is important in commercial micropropagation systems. For example, more elongated shoots of E. grandis and related hybrids often root more efficiently than less elongated shoots.

### EXAMPLE 7

### Propagation of eucalyptus with oxygenation by photosynthesis

The procedure described in Example 6 i.e. cultivation in the light and in the dark was carried out except that the gas distribution tubes were omitted from the apparatus. In this system there is no agitation other than by convection and/or diffusion, and no active i.e. externally applied oxygenation. The shoots were from the micropropagated E. grandis seedling line T14 L10.

The shoot multiplication rates obtained after 22 days were 6.2 in the vessel incubated in the light and 1 (no multiplication) in the dark. The result indicates that photosynthesis can compensate at least in part for the lack of active oxygenation i.e. externally applied oxygenation.

### EXAMPLE 8

### Propagation of eucalyptus with oxygenation and light

The method described in Example 6 using the continuous light conditions was repeated except that the air bubbled through the liquid medium was replaced by pure nitrogen. Shoots of E. grandis x camaldulensis clone 11/25 were used.

A 4-fold multiplication rate was obtained using the nitrogen, as compared to a multiplication rate of 10 when air is used (see Example 6). This indicates that adequate oxygenation, by active i.e. externally applied oxygenation and/or by photosynthesis, is required to support the multiplication of the shoots.

### EXAMPLE 9

### Propagation of eucalyptus using different inoculum sizes

The method described in Example 2 was carried out except that the vessels were inoculated with either 5, 20 or 40 shoots of E. grandis x camaldulensis clone 11/25. After 23 days incubation, multiplication rates obtained were 8, 24.7 and 36.4, respectively. This suggests that there is an advantage in using a high inoculation density.

### EXAMPLE 10

### Propagation of eucalyptus by recycling shoot material

Using the procedure described in Example 2, E. grandis x camaldulensis clone 11/25 and micropropagated seedling line T14 L10 were inoculated into the culture vessels and harvested after 23 days. 20 shoots were dissected from the shoot masses and immediately inoculated into vessels containing fresh liquid medium and incubated for a further 23 days. This process was repeated for one more cycle, giving a total of three propagation cycles.

The shoot multiplication rates did not differ significantly between cycles and there was no deterioration in the quality of the shoots produced after successive cycles. This demonstrates that shoot material can be recycled through successive rounds of liquid micropropagation, which is a significant commercial advantage.

### EXAMPLE 11

### Propagation of Acacia mangium

(i) Micropropagated cultures of Acacia mangium were established from seed (seedlot 945) obtained from HDRC, Ryton on Dunsmore, Coventry CV8 3LG UK.
(ii) Micropropagated Acacia mangium shoots previously produced on semi-solid medium comprising Complete MS salts Medium (Murashige and Skoog, 1962) supplemented with 2.2 µM BAP were inoculated into liquid medium of the same composition but lacking gelling agent. The method and apparatus were otherwise as described in Example 2.
   The shoots do not show signs of hyperhydricity (vitrification) and multiply well (multiplication rates of 12-15 fold/month). Shoot clumps form roots from the base. The shoot multiplication rate of this seedling-derived material using the method previously described on semi-solid medium is less 3 fold/month.
(iii) 10-20 mm long shoots produced using the liquid micropropagation described in section (ii) above were harvested, dipped in rooting powder (Seradix No. 2 rooting powder, Hortichem Ltd. Salisbury, Wilts SP2 7NU, UK) and set for rooting in compost in a fog chamber (96% relative humidity) at 25 °C under shaded daylight (maximum 250 µmol m⁻² s⁻¹). Rooting efficiencies obtained were greater than 70% for material produced in the process compared with less than 60% rooting for shoots produced on semi-solid medium using the same rooting procedure.
(iv) The effect of repeated cycling of Acacia mangium shoot material through successive rounds of liquid micropropagation was determined using the method described in section (ii) above. Shoots were harvested after an initial cycle of one month propagation in the air lift fermenter system described in section (ii) above, harvested and reinoculated into vessels containing fresh medium and cultured for a further month. This process was repeated for one more cycle, giving a total of three propagation cycles. The shoot multiplication rates did not differ significantly between cycles and there was no noticeable deterioration of the quality of the shoots produced after repeated cycling. The ability to recycle the starting material is a considerable commercial advantage.

### EXAMPLE 12

### Propagation of Olive

Micropropagated shoots of Olea europaea cultivar Dolce Agogia previously produced on semi-solid medium as described by Rugini et al. (Sci Hortic (Amst), 24(2), 1984, 123-134) were inoculated into liquid medium of the same composition but with the phytohormones adjusted to 4.9 µM Zeatin and 2.9µM GA₃ (gibberellic acid) and lacking gelling agent. The method and apparatus were otherwise as described in Example 2.

Very healthy material was obtained. Multiplication rates are slightly higher than any published for gelled media systems (7 fold/month in the liquid system vs a maximum of 6/month for this genotype on gelled media systems) and there were two further, important advantages of the liquid system over the gelled system:
1) The resulting shoots are much healthier; gelled systems all report chlorosis (yellowing) whereas the liquid system produces dark green, healthy shoots.
2) The shoots produced in the liquid system are more elongated and therefore do not require an extensive elongation step prior to rooting. Shoots produced on gelled media systems require a lengthy (more than 1 month) elongation step prior to rooting.

### EXAMPLE 13

### Propagation of Rhododendron

Micropropagated shoots of Rhododendron yakushimanum cultivar Dopey previously produced on semi-solid medium as described by Lloyd and McCowan (Combined Proceedings of the International Plant Breeders Society 30, 1980, 421-437) were inoculated into liquid medium of the same composition but with the phyto-hormones adjusted to 2.5 µM 2iP (N⁶-(2-isopentyl adenine) and lacking gelling agent. The method and apparatus were otherwise as described in Example 2.

6-fold multiplication was obtained in 4 weeks, compared with 2-3 fold multiplication in 6 weeks on gelled medium.

The uniformity of the shoots obtained according to the liquid culture system was excellent and far superior to that obtained using gelled propagation systems. One major advantage of the liquid system is that no adventitious shoots are produced and all new shoots are produced from axillary buds. In all the gelled systems, large amounts of callus can be formed on the base of the multiplying shoots. Adventitious shoots are often produced from this callus via organogenesis. These adventitious shoots are sometimes not true to type (i.e. are abnormal) due to the phenomenon of somaclonal variation. (This phenomenon is often associated with changes in chromosome number or chromosomal rearrangements.) Currently, commercial Rhododendron producers have to sacrifice multiplication rates in order to avoid callus formation and adventitious shoot formation to ensure that the plants they are propagating are true to type. The liquid system of the present invention avoids this problem.

Shoots produced in the bioreactor were elongated by 2-3 weeks culture on the semi-solid medium as described above prior to rooting into compost using the method as described for Acacia mangium. Rooting efficiencies obtained were in excess of 90% and equivalent to rooting efficiencies of shoots produced on semi-solid medium as previously described, using the same rooting procedure. Manipulation of the growth conditions in the bioreactor will facilitate the production of more elongated shoots which will be suitable for setting for rooting immediately after harvest from liquid culture.

### EXAMPLE 14

### Propagation of Malus domestica

Micropropagated Malus domestica cultivar Greensleeves shoots previously produced on semi-solid medium comprising complete MS salts Medium (Murashige and Skoog, 1962) supplemented with 5 µM BAP, 0.5 µM IBA and 3µM GA₃ were inoculated into liquid medium of the same composition but lacking both gelling agent and plant growth regulators, the method and apparatus otherwise being described in Example 2.

Shoot multiplication rates achieved were 3/month, the same as that obtained on semi-solid medium described above. However, the liquid system requires fewer handling steps than the semi-solid system, so has commercial advantages. The shoots obtained showed some symptoms of hyperhydricity but when shoots harvested from the vessel were transferred to semi-solid medium as described above, all new growth was free of symptoms.

### EXAMPLE 15

### Propagation of Forsythia

15 micropropagated shoots of Forsythia x intermedia cv. Lynwood previously produced on semi-solid medium LS medium (Linsmaier ES & Scoog S, Phys. Pl. (1965) 18 100-127) supplemented with 3% sucrose and 10 µM BAP were inoculated into liquid medium of the same composition but lacking gelling agent, the method and apparatus otherwise being described in Example 2. The shoots were harvested after four weeks.

The harvested shoots had grown well. There were no signs of hyperhydricity and the shoots were similar in appearance, in particular in texture and colour, to shoots grown on the semi-solid medium. However, on the semi-solid medium a clump of many short shoots may develop, of which only 2-3 shoots normally elongate. In the liquid medium all the shoots had elongated, indicating that overall multiplication rates may be considerably higher than on the semi-solid medium. The shoots harvested from the liquid medium appeared to be suitable for rooting directly into compost under fog in a greenhouse.

### EXAMPLE 16

### Propagation of Syringia

Semi-solid LS medium (Linsmaier ES & Scoog S, Phys. P1. (1965) 18 100-127) supplemented with 3% sucrose and 30 µM BAP or with 3% sucrose and 10µM zeatin were used for the micropropagation of shoots of Syringia vulgaris cv. Madame Lamoine. The BAP-supplemented medium gives good proliferation but poor elongation while the zeatin medium gives poor shoot proliferation. Clumps of shoots produced on the BAP medium were transferred to the zeatin medium for elongation. For liquid culture the BAP medium without the gelling agent was used as the liquid medium.

15 Micropropagated shoots were inoculated into the liquid medium, the method and apparatus otherwise being described in Example 2. The shoots were harvested after four weeks. The original explant leaves had turned brown but new growth was dark green and showed few symptoms of hyperhydricity. The shoots appear to be suitable for rooting directly into compost under fog in a greenhouse.

## Claims

1. A process for the micropropagation of shoots, rooted shoots or seedlings of a woody plant, which comprises cultivating the shoots, rooted shoots or seedlings in an oxygenated liquid medium, the shoots, rooted shoots or seedlings being submerged in the liquid medium.

2. A process as claimed in claim 1, wherein the liquid medium is agitated.

3. A process as claimed in claim 2, wherein the culture medium is agitated and oxygen is provided by passing oxygen or air through the medium.

4. A process as claimed in any one of claims 1 to 3, wherein the shoots, rooted shoots or seedlings are free to move in the liquid medium.

5. A process as claimed in any one of claims 1 to 3, wherein the movement of the shoots, rooted shoots or seedlings is restricted.

6. A process as claimed in any one of claims 1 to 5, wherein the shoots, rooted shoots or seedlings are obtained from a cultivar, clone or seed.

7. A process as claimed in any one of claims 1 to 6, wherein the woody plant is a gymnosperm or dicotyledenous angiosperm used for wood pulp, for fuel or for timber; a tree, shrub or bush that produces fruit or nuts; a tree or shrub from which a commercially useful product other than a fruit or nut is obtained; or an ornamental tree or shrub.

8. A process as claimed in any one of claims 1 to 6, wherein the woody plant is of a sclerophyllous species.

9. A process as claimed in claim 8, where the woody plant is a Rhododendron, Azalea or Kalmia (Ericaceae); an Olea (Oleaceae); or an Australian Acacia.

10. A process as claimed in any one of claims 1 to 6, wherein the woody plant is a Malus (apple); Pyrus, Prunus or Rosa (Rosaceae); Forsythia or Syringia (Oleaceae).

11. A process as claimed in any one of claims 1 to 6, wherein the woody plant is a eucalypt.

12. A process as claimed in claim 11, wherein the eucalypt is a eucalypt of the sub-genus Eucalyptus symphyomyrtus.

13. A process as claimed in claim 12, wherein the eucalypt is E. grandis, E. globulus, E. nitens, E. dunnii, E. saligna, E. camaldulensis, E. urophylla or a hybrid thereof, or E. regnans, E. citriodora, E. fraxinoides or a hybrid thereof.

14. A process as claimed in any one of claims 1 to 13, wherein cultivation of shoots is continued until rooting has initiated or has taken place.

15. A process as claimed in any one of claims 1 to 14, wherein a resulting shoot, rooted shoot or seedling is further micropropagated by repeating the process as claimed in any one of claims 1 to 5.

16. A process as claimed in any one of claims 1 to 15, wherein the oxygenated liquid medium also comprises an antibiotic.

17. A process as claimed in any one of claims 1 to 16, wherein the resulting shoots, rooted shoots or seedlings are grown into a plant.

18. A process as claimed in claim 17, wherein wood pulp, fuel, timber, fruit, nuts, a pharmaceutically suitable substance or a precursor for a pharmaceutically suitable substance is obtained from the plant.

## Patentansprüche

1. Verfahren zur Mikrovermehrung von Schößlingen, bewurzelten Schößlingen oder Sämlingen einer holzigen Pflanze, das ein Züchten der Schößlinge, bewurzelten Schößlinge oder Sämlinge in einem sauerstoffhältigen flüssigen Medium umfaßt, wobei die Schößlinge, bewurzelten Schößlinge oder Sämlinge in dem flüssigen Medium submers gehalten werden.

2. Verfahren nach Anspruch 1, worin das flüssige Medium in Bewegung gehalten wird.

3. Verfahren nach Anspruch 2, worin das Kulturmedium in Bewegung gehalten wird und Sauerstoff durch Durchleiten von Sauerstoff oder Luft durch das Medium zur Verfügung gestellt wird.

4. Verfahren nach einem der Ansprüche 1 bis 3, worin die Schößlinge, bewurzelten Schößlinge oder Sämlinge in dem flüssigen Medium frei beweglich sind.

5. Verfahren nach einem der Ansprüche 1 bis 3, worin die Bewegung der Schößlinge, bewurzelten Schößlinge oder Sämlinge begrenzt ist.

6. Verfahren nach einem der Ansprüche 1 bis 5, worin die Schößlinge, bewurzelten Schößlinge oder Sämlinge von einer Kultursorte, einem Klon oder einem Samen erhalten werden.

7. Verfahren nach einem der Ansprüche 1 bis 6, worin die holzige Pflanze eine für Holzzellstoff, für Brennstoff oder für Bauholz verwendete Gymnosperme oder zweikeimblättrige Angiosperme ist; ein Früchte oder Nüsse produzierender Baum, Strauch oder Busch ist; ein Baum oder Strauch ist, von dem ein anderes kommerziell brauchbares Produkt als eine Frucht oder eine Nuß erhalten wird; oder ein Zierbaum oder Zierstrauch ist.

8. Verfahren nach einem der Ansprüche 1 bis 6, worin die holzige Pflanze von einer Sklerophyllenspezies ist.

9. Verfahren nach Anspruch 8, worin die holzige Pflanze ein Rhododendron, eine Azalee oder eine Lorbeerrose (Ericaceae); eine Olea (Oleaceae); oder eine australische Akazie ist.

10. Verfahren nach einem der Ansprüche 1 bis 6, worin die holzige Pflanze ein Malus (Apfel); Pyrus, Prunus oder Rosa (Rosaceae); Forsythia oder Syringia (Oleaceae) ist.

11. Verfahren nach einem der Ansprüche 1 bis 6, worin die holzige Pflanze ein Eukalyptus ist.

12. Verfahren nach Anspruch 11, worin der Eukalyptus ein Eukalyptus der Untergattung Eukalyptus symphyomyrtus ist.

13. Verfahren nach Anspruch 12, worin der Eukalyptus E. grandis, E. globulus, E. nitens, E. dunnii, E. saligna, E. camaldulensis, E. urophylla oder eine Hybride hievon oder E. regnans, E. citriodora, E. fraxinoides oder eine Hydride hievon ist.

14. Verfahren nach einem der Ansprüche 1 bis 13, worin das Züchten von Schößlingen fortgesetzt wird, bis das Bewurzeln initiiert oder erfolgt ist.

15. Verfahren nach einem der Ansprüche 1 bis 14, worin ein erhaltener Schößling, bewurzelter Schößling oder Sämling durch Wiederholen des Verfahrens nach einem der Ansprüche 1 bis 5 weiter mikrovermehrt wird.

16. Verfahren nach einem der Ansprüche 1 bis 15, worin das sauerstoffhältige flüssige Medium auch ein Antibiotikum umfaßt.

17. Verfahren nach einem der Ansprüche 1 bis 16, worin die gebildeten Schößlinge, bewurzelten Schößlinge oder Sämlinge zu einer Pflanze gezogen werden.

18. Verfahren nach Anspruch 17, worin aus der Pflanze Holzzellstoff, Brennstoff, Bauholz, Früchte, Nüsse, eine pharmazeutisch nützliche Substanz oder ein Vorläufer für eine pharmazeutisch nützliche Substanz gewonnen werden.

## Revendications

1. Procédé pour la multiplication de pousses, pousses enracinées ou jeunes plants d'une plante ligneuse, qui comprend la culture des pousses, pousses enracinées ou jeunes plants dans un milieu liquide oxygéné, les pousses, pousses enracinées ou jeunes plants étant immergés dans le milieu liquide.

2. Procédé suivant la revendication 1, dans lequel le milieu liquide est agité.

3. Procédé suivant la revendication 2, dans lequel le milieu de culture est agité et de l'oxygène est amené en faisant passer de l'oxygène ou de l'air à travers le milieu.

4. Procédé suivant l'une quelconque des revendications 1 à 3, dans lequel les pousses, pousses enracinées ou jeunes plants sont libres de bouger dans le milieu liquide.

5. Procédé suivant l'une quelconque des revendications 1 à 3, dans lequel le mouvement des pousses, pousses enracinées ou jeunes plants est limité.

6. Procédé suivant l'une quelconque des revendications 1 à 5, dans lequel les pousses, pousses enracinées ou jeunes plants sont obtenus à partir d'un cultivar, d'un clone ou d'une semence.

7. Procédé suivant l'une quelconque des revendications 1 à 6, dans lequel la plante ligneuse est un gymnosperme ou un angiosperme dicotylédone utilisé pour de la pâte de bois, pour du combustible ou pour du bois d'oeuvre, un arbre, un arbrisseau ou une basse tige qui produit des fruits ou des noix, un arbre ou arbrisseau d'où l'on obtient un produit intéressant du point de vue commercial autre qu'un fruit ou une nuit, ou un arbre ou arbrisseau ornemental.

8. Procédé suivant l'une quelconque des revendications 1 à 6, dans lequel la plante ligneuse est d'une espèce sclérophylle.

9. Procédé suivant la revendication 8, dans lequel la plante ligneuse est un Rhododendron, Azalea ou Kalmia (éricacées), un Olea (oléacées) ou un Acacia d'Australie.

10. Procédé suivant l'une quelconque des revendications 1 à 6, dans lequel la plante ligneuse est un Malus (pomme), Pyrus, Prunus ou Rosa (rosacées), Forsythia ou Syringia (oléacées).

11. Procédé suivant l'une quelconque des revendications 1 à 6, dans lequel la plante ligneuse est un eucalyptus.

12. Procédé suivant la revendication 11, dans lequel l'eucalyptus est un eucalyptus du sous-genre Eucalyptus symphyomyrtus.

13. Procédé suivant la revendication 12, dans lequel l'eucalyptus est E. grandis, E. globulus, E. nitens, E. dunnii, E. saligna, E. camaldulensis, E. urophylla ou un hybride de ceux-ci ou bien E. regnans, E. citriodora, E. fraxinoides ou un hybride de ceux-ci.

14. Procédé suivant l'une quelconque des revendications 1 à 13, dans lequel la culture des pousses est poursuivie jusqu'à ce que l'enracinement ait commencé ou ait pris place.

15. Procédé suivant l'une quelconque des revendications 1 à 14, dans lequel une pousse, une pousse enracinée ou un jeune plant résultant est à nouveau micromultiplié en répétant le procédé suivant l'une quelconque des revendications 1 à 5.

16. Procédé suivant l'une quelconque des revendications 1 à 15, dans lequel le milieu liquide oxygéné comprend également un antibiotique.

17. Procédé suivant l'une quelconque des revendications 1 à 16, dans lequel les pousses, pousses enracinées ou jeunes plants résultants sont amenés à croître sous la forme d'une plante.

18. Procédé suivant la revendication 17, dans lequel de la pâte de bois, du combustible, du bois d'oeuvre, un fruit, des noix, une substance pharmaceutiquement appropriée ou un précurseur pour une substance pharmaceutiquement appropriée sont obtenus à partir de la plante.
